Europäisches Patentamt

European Patent Office

Office européen des brevets

(19)

(11) **EP 1 106 625 A1**

(12) ## EUROPEAN PATENT APPLICATION

(43) Date of publication:
**13.06.2001 Bulletin 2001/24**

(51) Int Cl.[7]: **C07K 7/06**, A61K 38/08,
G01N 33/53, C12N 15/11,
A61K 31/7088, C12N 1/21

(21) Application number: **99122858.6**

(22) Date of filing: **17.11.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant: **ZLB Bioplasma AG
3000 Bern 22 (CH)**

(72) Inventors:
• **Miescher, Silvia
3004 Bern (CH)**
• **Hofmann, Andreas
3172 Niederwangen (CH)**

• **Fisch, Igor
1208 Geneva (CH)**

(74) Representative:
**Weiss, Wolfgang, Dipl.-Chem. Dr. et al
Weickmann & Weickmann
Patentanwälte
Postfach 86 08 20
81635 München (DE)**

Remarks:
The applicant has subsequently filed a sequence listing and declared, that it includes no new matter.

(54) **Rhesus D specific peptide sequences**

(57)   The invention relates to novel peptides capable of binding to anti-Rhesus D antibodies and recombinant polypeptides comprising said peptide sequences. Further, a method for identifying peptides capable of binding to anti-Rhesus D antibodies is disclosed.

EP 1 106 625 A1

**Description**

The invention relates to novel peptides capable of binding to anti-Rhesus D antibodies and recombinant polypeptides comprising said peptide sequences. Further, a method for identifying peptides capable of binding to anti-Rhesus D antibodies is disclosed.

Hemolytic disease of the newborn (HDN) is the general designation for hemolytic anemia of fetuses and newborn babies caused by antibodies of the mother. These antibodies are directed against antigens on the surface of the fetal erythrocytes. These antigens can belong to the Rhesus, ABO or other blood group systems.

The Rhesus blood group system includes 5 major antigens: D, C, c, E and e (Issit, P.D., Med. Lab. Sci. 45 (1988), 395). The D antigen is the most important of these antigens as it is highly immunogenic eliciting anti-Rhesus D antibodies during Rhesus incompatible pregnancies and following transfusion of Rhesus incompatible blood. The D antigen is found in approximately 85% of Caucasians in Europe and those individuals are said to be Rhesus positive. Individuals lacking the D antigen are called Rhesus negative. The expression of the D antigen can vary due to either low antigen density, hereafter known as D or $D^u$, or due to partial antigenicity, hereafter known as partial D antigens.

The Rhesus D antigen, a membrane protein of the erythrocyte, has recently been cloned and its primary structure described (Le Van Kim, C. et al. PNAS 89 (1992), 10925). Modelling studies suggest that the Rhesus D antigen has 12 transmembrane domains with only very short connecting regions extending outside the cell membrane or protruding into the cytoplasm.

The partial D phenotypes were first identified in people who carried D antigen on their red cell but who had an alloanti-D in their sera (Rose, R.R. and Sanger, R., Blood groups in man, Blackwell Scientific, Oxford, U.K. (1975); Tippett, P. et al., Vox Sanguinis 70 (1996), 123). This can be explained by regarding the D antigen as a mosaic structure with at least 9 different epitopes (epD1 to epD9). Thus in some D variant people the red cells lack part of this mosaic and antibodies are made to the missing D epitopes. Rhesus positive individuals that make antibodies against partial D antigens have been classified into six main different categories ($D^{II}$ to $D^{VII}$) each having a different abnormality in the D antigen. More recently it has been shown that these D categories gave different patterns of reactions when tested against panels of human monoclonal anti-D antibodies (Tippett, P. et al., Vox Sanguinis 70 (1996), 123). The different reaction patterns identified the 9 epitopes and so define the different partial D categories. The number of epitopes present on the D antigen varies from one partial D category to another with the $D^{VI}$ category expressing the least, epD3, 4 and 9. The $D^{VI}$ category is clinically important as a $D^{VI}$ woman can be immunized strongly enough to cause hemolytic disease of the newborn.

The prophylatic efficacy of anti-RhD IgG for prevention of hemolytic disease of the newborn is well established and has been in routine use for many years. As a result this severe disease has become a rarity. Nevertheless the underlying cause of the disease, i.e. RhD incompatibility between a RhD negative mother carrying a RhD positive child still remains and thus requires a continual supply of therapeutic anti-RhD IgG.

In recent years the assurance of a continual supply of anti-RhD IgG has become an increasing problem. The pool of available hyperimmune serum from alloimmunized mulitparous Rhesus negative women has drastically decreased due to the success of prophylactic anti-RhD administration. Thus the current methods of production require repeated immunization of an increasingly reluctant pool of donors for the production of high titer antiserum (Selinger, M., Br. J. Obstet. Gynaecol 98 (1991), 509). There are also associated risk factors and technical problems such as the use of Rhesus positive red blood cells for repeated immunization carrying the risk of transmission of viral diseases like hepatitis B, AIDS and other as yet unknown viruses (Hughes-Jones, N.C., Br. J. Haematol. 70 (1988), 263). Therefore an alternative method for production of anti-RhD antibodies is required.

A series of anti-Rhesus D antibodies have been selected from two human recombinant antibody libraries using the phage display system (WO97/49809; Miescher et al., Vox Sanguinis 75 (1998), 278-287). Some of these antibodies have been selected for development as recombinant full length IgG1 and/or IgG3 antibodies for prophylaxis and prevention of HDN. Recombinant antibodies are currently in development, however, it is not known whether a recombinant monoclonal antibody can replace the polyclonal antibody preparations of the prior art. Thus, there is a need for alternative methods of immunizing donors, which avoid immunization with foreign erythrocytes.

According to the present invention it was found that peptide sequences derived from different random peptide libraries displayed on phage particles bind specifically to anti-Rhesus D antibodies. These peptide sequences can mimic the Rhesus D antigen and may be employed for the manufacture of a synthetic immunogen which may replace the current immunization with foreign erythrocytes. Further, the peptide sequences may be used for the characterization of the binding site of anti-Rhesus D antibodies in order to aid the registration of human recombinant antibodies for therapy.

Subject matter of the present invention are peptides capable of binding to Rhesus D antibodies, e.g. the monoclonal anti-Rhesus D antibody known as IgG1-3 or the polyclonal antiserum Rhophylac® (ZLB, Central Laboratory, Swiss Red Cross).

The peptides of the invention comprise:

(a)

    (i) the amino acid sequence (I):

<div align="center">

## GGMVDVYNGR

</div>

    (ii) the amino acid sequence (I) wherein up to 3 amino acids are replaced by different amino acids or
    (iii) an amino acid sequence which comprises at least 6 consecutive amino acids from (I);

(b)

    (i) the amino acid sequence (II):

<div align="center">

## MRYDYDILM

</div>

    (ii) the amino acid sequence (II) wherein up to 3 amino acids are replaced by different amino acids or
    (iii) an amino acid sequence which comprises at least 6 consecutive amino acids from (II);

(c)

    (i) the amino acid sequence (III):

<div align="center">

## KGFWDREWGL

</div>

    (ii) the amino acid sequence (III) wherein up to 3 amino acids are replaced by different amino acids or
    (iii) an amino acid sequence which comprises at least 6 consecutive amino acids from (III);

(d)

    (i) the amino acid sequence (IV):

<div align="center">

## GYWSAKẀAVK

</div>

    (ii) the amino acid sequence (IV) wherein up to 3 amino acids are replaced by different amino acids or
    (iii) an amino acid sequence which comprises at least 6 consecutive amino acids from (IV);

(e)

    (i) the amino acid sequence (V):

<div align="center">

## YFFPRGSGM

</div>

    (ii) the amino acid sequence (V) wherein up to 3 amino acids are replaced by different amino acids or
    (iii) an amino acid sequence which comprises at least 6 consecutive amino acids from (V);

(f)

    (i) the amino acid sequence (VI):

WHLSP

(ii) the amino acid sequence (VII):

RHLSP

or
(iii) an amino acid sequence which comprises at least 4 consecutive amino acids from (VI) or (VII).

(g)

(i) the amino acid sequence (VIII):

QLIVMW

(ii) the amino acid sequence (IX):

RLIVMW

or
(iii) an amino acid sequence which comprises at least 4 consecutive amino acids from (VIII) or (IX)

Preferably, the peptides have lengths from 5-20 amino acids, more preferably from 5-12 amino acids. The peptides may be linear or circular, e.g. preferably connected by Cys-Cys bridges. Further, the peptides may be N- and/ or C-terminally Modified, e.g. by N-terminal acylation and/or by C-terminal amidation. Besides, natural amino acids, the peptides may comprise further moieties, e.g. non-natural amino acids, linker groups or spacer groups.

Apart from the concretely identified peptide sequences (I) to (IX), the present invention also concerns variant and partial sequences thereof, e.g. peptides on the basis of amino acid sequences (I) to (V) wherein up to three, preferably, up to two and most preferably up to one amino acid is/are replaced by different amino acids. Such amino acid substitutions are preferably conservative substitutions, i.e. an amino acid with hydrophobic side chain is replaced by another amino acid with hydrophobic side chain, an amino acid with hydrophilic side chain is replaced by another amino acid with hydrophilic side chain and/or an amino acid with charged side chain is replaced by another amino acid with a side chain carrying the same charge. A further example of partial sequences are peptides derived from amino acids (I) to (V), containing at least six, preferably at least seven and most preferably at least eight consecutive amino acids of the respective basic sequence or peptides derived from amino acid sequences (VI) and (IX) which comprise at least four consecutive amino acids of the respective basic sequence. It should be noted that the peptides as defined above may be used separately or in multiple form, e.g. several different peptides together either in parallel or in series.

The peptides of the present invention may be obtained by chemical synthesis according to known methods, e.g. the Merrifield method. Alternatively, the peptides may be produced by recombinant DNA technology, particularly if they are genetically fused to a recombinant polypeptide.

In order to enhance the immunogenicity of the peptides, they may be incorporated into vesicles or coupled to a carrier molecule, e.g. a macromolecular carrier or to a solid phase. An example for a suitable vesicle are liposomes (Guan et al., Bioconjugate Chemistry 9 (1998), 451-458). The coupling to a carrier or a solid phase may be accomplished by chemical reaction, i.e. coupling a reactive group on the peptide with a corresponding group on the carrier or the solid phase. In this embodiment of the invention, the carrier may be a proteinaceous material, e.g. a protein which is known to be suitable as carrier of immunogenic peptides, e.g. albumin or keyhole limpet hemocyanine (KLH), or a non-proteinaceous macromolecule, e.g. a carbohydrate such as dextran. Further examples of suitable carriers are multiple antigen peptides which are synthetic macromolecules based on a peptide core matrix containing trivalent amino acid residues, e.g. lysine, to which the desired peptide sequences are coupled in absence of a carrier protein (Nardin et al., Adv. Immunol. 60 (1995), 105-149) or polyoxime carriers (Rose et al., Peptides: Chemistry, Structure and Biology, 1996; Nardin et al., Vaccine 16 (1998), 590-600). The carrier may comprise at least one of the peptide sequences as defined above and preferably several of the peptide sequences which may be the same or different, e.g. up to ten of such peptide sequences. Examples for solid phases are reaction vessels, e.g. for diagnostic applications or chromatographic matrix materials, e.g. beads for preparative or therapeutical applications.

In a preferred embodiment the peptides are genetically fused to a recombinant polypeptide. The recombinant polypeptide may be a recombinant phage polypeptide, e.g. a polypeptide from a filamentous phage, e.g. pIII. These recombinant polypeptides of the present invention are suitable for phage display applications, e.g. to identify novel antibody sequences capable of binding to Rhesus D antigens or to characterize the binding properties of known antibodies. Further, the recombinant polypeptide may be a pharmaceutically acceptable fusion polypeptide, which may be used as an immunogen for therapeutic or prophylactic applications or as a diagnostic agent. Specific examples for pharmaceutically acceptable polypeptides are e.g. the albumin binding protein (ABP) (Baumann et al., J. Immunol. Meth. 221 (1998), 95-106).

Preferably, the peptides are fused to the N- and/or C-terminus of the recombinant polypeptide sequences. The recombinant polypeptide may contain one or several peptide sequences, which may be the same or different.

A further subject matter of the present invention is a nucleic acid encoding the peptide or polypeptide as described above and a vector comprising at least one copy of the nucleic acid, preferably in operative linkage to an expression control sequence. Further, the invention refers to a cell comprising the nucleic acid or the vector as described above. The cell may be a prokaryotic cell, e.g. an E. coli cell or a eukaryotic cell, e.g. a mammalian cell. The peptide or polypeptide may be prepared by culturing the cell under suitable conditions, wherein the peptide or polypeptide is produced and the peptide or polypeptide is obtained from the cell or the culture medium. Examples for suitable expression systems for the peptides or polypeptides of the present invention are described, e.g. by Sambrook et al., Molecular Cloning, A Laboratory Manual (1998), Cold Spring Harbour Laboratory Press.

The peptides and polypeptides of the present invention are suitable for pharmaceutical purposes. Thus, a pharmaceutical composition is provided comprising as an active ingredient at least one peptide or polypeptide as described above optionally together with pharmaceutically acceptable carriers, diluents and adjuvants. The composition may be a diagnostic or therapeutic compositon. More preferably, the composition is an immunogenic composition suitable for injection.

The peptides and polypeptides may be used for the manufacture of an agent for the diagnosis, therapy or prophylaxis of diseases associated with Rhesus D antigen. Preferably, the disease is hemolytic disease of the newborn (HDN). An example of another disease is idiopathic thrombocytopenic purpura (ITP).

The peptides coupled to a suitable solid phase may be used as affinity reagents in order to purify antibodies or antibody fragments binding thereto. E.g. anti-Rhesus D antibodies may be purified from hyperimmune plasma either directly or in combination with other methods. Further, the peptides are suitable for affinity chromatography in order to remove unwanted anti-Rhesus D antibodies from antibody preparations, e.g. polishing of IgG preparations such as Sandoglobulin, or from body fluids, e.g. removing unwanted anti-Rhesus D antibodies from plasma of patients with autoimmune hemolytic anemia (AIHA).

Nucleic acids or vectors encoding the peptides or polypeptides of the present invention are also suitable for pharmaceutical applications. E.g. DNA vaccines including nucleic acid sequences encoding the peptides optionally fused to a carrier protein may be constructed in a plasmid and administered to a subject in need thereof according to known methods (Donnelly et al., Annu. Rev. Immunol. 15 (1997), 617-648).

Further, the present invention relates to a method for identifying peptide sequences having immunologic properties of Rhesus D protein epitopes comprising subjecting an antibody or an antibody fragment, i.e. a part of an antibody comprising at least the antigen binding site, e.g. a Fab fragment, recognizing an epitope of Rhesus D protein to several panning rounds with a phage display library and identifying immunogenic peptide sequences wherein said peptide sequences are mimotopes which differ in their amino acid sequence from the amino acid sequence of Rhesus D protein. The antibody may be a monoclonal antibody such as IgG1-3 or a fragment thereof. Alternatively, polyclonal antisera such as the commercial product Rhophylac® may be used. The phage display library contains random peptides having a length of preferably six to sixteen amino acids, more preferably from eight to twelve amino acids. The peptides may be presented in a linear or in a cyclic form.

Further, the invention relates to peptides having immunological properties of Rhesus D protein epitopes which differ in their amino acid sequence from the amino acid sequence of Rhesus D protein. These peptides may be obtained by the method as described above. The peptides preferably have a length of five to twenty amino acids and may be coupled to a carrier as described above.

Preferably the mimotopes have a sequence identity of 70% or less, more preferably of 50% or less to the amino acid sequence of human Rhesus D protein. Sequence identity I is defined according to the equation:

$$I = \frac{n}{L} \times 100$$

wherein

L is the length of the peptide sequence and
n is the number of amino acid difference between peptide sequence and Rhesus D protein sequence.

Further, the invention shall be explained by the following figures and examples.

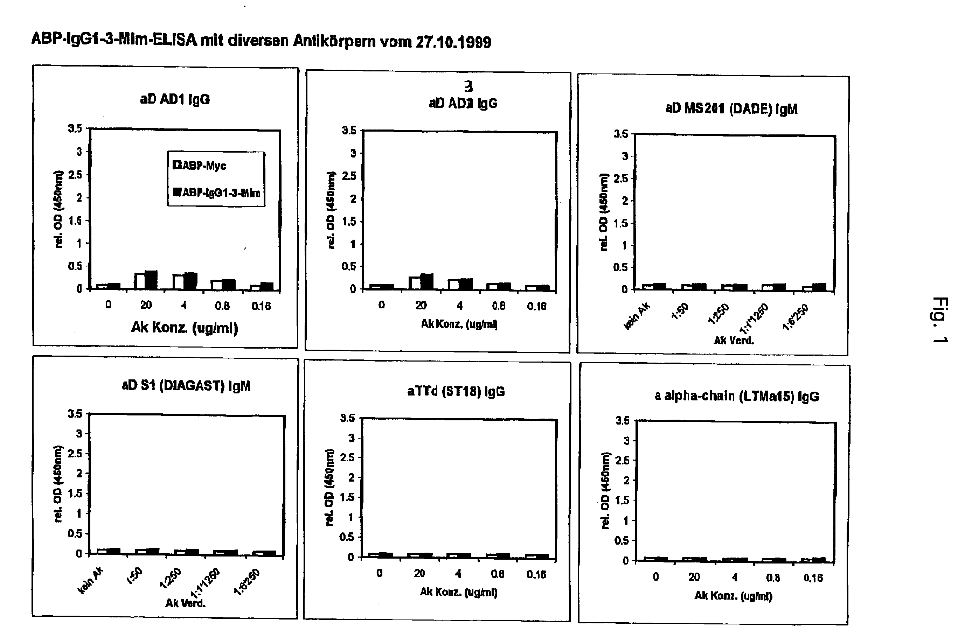

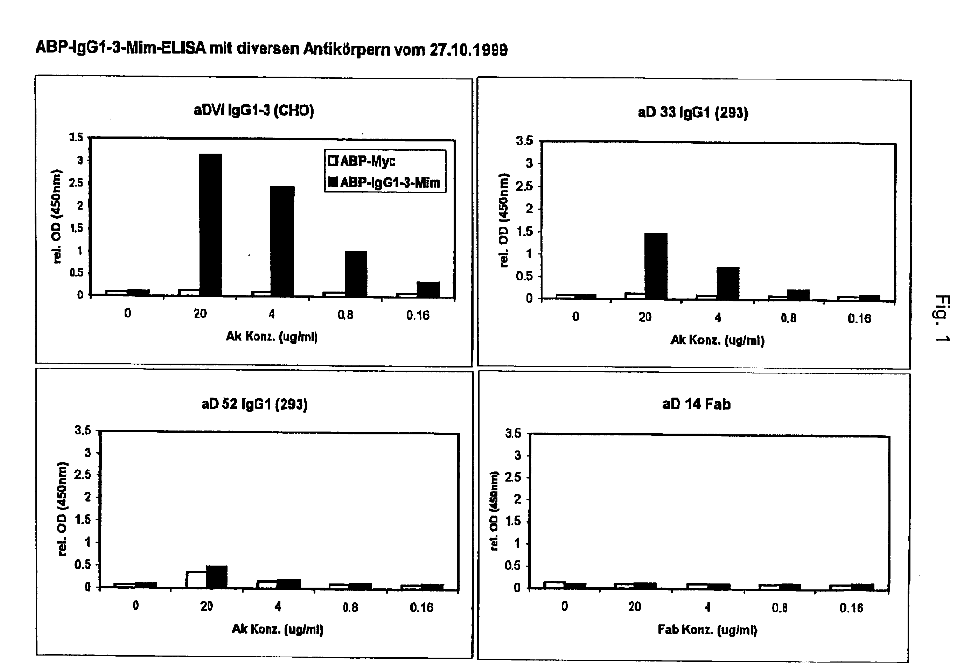

Fig. 1    shows the reactivity of a ABP-peptide (I) fusion polypeptide with different antibodies.

Example 1

1. Materials

The monoclonal anti-Rhesus D antibody known as lgG1-3 is described in WO97/49809. The sequence of the variable region of the Fab fragment is also deposited at the EMBL data bank as clone LD-6-3 (VH: Accession No. AJ 005977; VL: Accession No. AJ 005978). This antibody is under development as a potential recombinant therapeutic antibody. A purified antibody preparation was used. For comparative purposes a current polyclonal product, known as Rhophylac® (ZLB, SRK) was screened.

Circular decapeptide, linear nonapeptide and linear hexapeptide libraries were manufactured as described in Mazuchelli et al. (Blood 93 (1999), 1738-1748; Cwirla et al. (Proc. Natl. Acad. Sci. USA 87 (1990), 6378) and Burrit et al. (Anal. Biochem. 238 (1996), 1). Random peptides were expressed as fusion proteins on the pill protein. The Escherichia coli strain K91 was obtained from Stratagene (Zurich, Switzerland). The E. coli vector system used for the production of peptides fused to albumin binding protein (ABP) and a 6 x His tag was as described in Baumann et al. (Supra).

2. Methods

2.1 Panning procedure

EIA/RIA plates (#3690, Integra Biosciences, Wallisellen, Switzerland) were coated with anti-IgG1-3 Ab at a concentration of 20 μg/ml in 50 μl/well in phosphate buffered saline (PBS) buffer overnight at 4°C. A total of 4 wells were coated. Wells were blocked with PBS-3% bovine serum albumin (BSA) for 2 h at 37°C. Phage input of original library was 10 μl diluted in 200 μl PBS-3% BSA and distributed into 4 x 50 μl/well. Wells were incubated for 2 h at room temperature followed by washing 3 times in PBS, 150 μl/well, 5 x 5 min in PBS + 0.1 % Tween, 150 μl/well and 3 x PBS.150 μl/well. Specific phages were eluted by low (200 μl 0.1 M glycin-HCl pH 2.2) and successive high pH (200 μl 100 mM triethylamine). Each eluate was separately neutralised with 13 μl 2 M Tris buffer (13 μl/200 μl eluate). Both elution approaches were performed in quadruplicates and phages were pooled from these four wells. Eluted phages were amplified in E. coli K91 according to a standard procedure and used for the subsequent panning round.

During the panning rounds the titre of increasingly specific phages was assessed by the increase in colony forming units (cfu), and an immunobinding dot assay, followed by selection of individual clones, repeat of the immunobinding dot assay on a clonal level and selection of clones for extraction of DNA from bacterial plasmids and sequence analysis. For other antibodies basically the same procedure was used.

2.2 Assay for detection of Phage reactivity by immunobinding dot assay

The panning antigen (lgG1-3) and different controls diluted in PBS buffer were dotted onto nitrocellulose strips, air dried and blocked for 2 h at room temperature in PBS/casein (0.15%). Selected phages in 3 fold dilutions in PBS/casein were incubated overnight at room temperature, followed by washing 3 times 10 min in PBS. Then the developing antibody, (e.g. rabbit anti-phage antibody 1/1000 in PBS/casein) was incubated for 2 h at room temperature, then washed 3 x 5 min in PBS followed by developing the reaction with chloronaphthol substrate.

2.3 DNA extraction from bacterial plasmids

Selected clones were grown in E. coli K91 and the DNA was extracted using commercial kits according to the manufacturer's instructions.

2.4 Sequence analysis

DNA sequencing was performed on an ABI 373 A sequencing system. A primer was synthesized (5' gtt ttg tcg tct ttc cag acg 3') for detecting the random peptide sequences in the vector M13KBST X1 (Burrit J.B. et al., (1995) J. Biol. Chem. 270, 16974).

2.5 Transfer of peptides from phages to a fusion protein

The selected peptides were cloned in a vector system containing ABP linked to a 6 x His tag (Baumann et al., Supra).

The ABP vector pSB 411 was digested with Sfil. DNA was phenol extracted, ethanol precipitated and the open vector was separated on 0.8% agarose gel and excised. The vector was extracted using a Quiagen DNA Extraction kit and annealed oligonucleotides encoding the desired peptide sequences were ligated.

E. coli XL-1 blue cells were $CaCl_2$ transformed and plasmid DNA from single colonies obtained after the transformation was prepared and analysed for correspondance of the sequence insert to the original selected peptide sequence.

Bacterial colonies containing the correct inserts were grown to an OD (600 nm)= 1 in Super Broth medium supplemented with 25 µg/ml chloramphenicol at 30°C. Cells were induced with 1 mM isopropyl-β-D-thiogalactopyranoside (IPTG) and incubated for four hours. After centrifugation, cells were sonicated and frozen and fusion proteins were affinity purified by the 6 x His tag using TALON® Ni-NTA resin (Clontech, Palo Alto, U.S.A.) The ABP fusion protein was eluted with 500 nM imidazole. Fusion proteins were analysed on a Coomassie stained nonreducing 15% polyacrylamide SDS gel. Immunoblots were performed with a peroxidase-labelled anti 6 x His antibody (Qiagen, Basel, Switzerland).

2.6 ELISA assay for testing activity and specifity of ABP fusion proteins

Briefly, a standard ELISA procedure was used where the ABP+ peptide fusion protein was immobilized on EIA half well plates followed by incubation with a range of different test and control antibodies to test specificity. Specific binding of the different antibodies was detected using anti-human IgG or IgM or anti-human Fab antibodies, respectively labeled with peroxidase, followed by incubation with the specific substrate tetramethylbenzidine (TMB). Absorbance values were measured with an ELISA reader at 450 nm.

3. Results

3.1 Sequence analysis

The sequences that showed a consensus and evidence of specific reactivity either expressed on the phage or transferred to the ABP fusion protein and whether derived from the cyclic or the linear random peptide libraries are given in Table 1 below. The sequences show no identity to the sequence of the Rh D protein and are therefore considered as mimotopes rather than epitopes and probably indicate that the anti-RhD antibodies recognise a conformational determinant on the Rh D protein.

Table 1

| Results of the Sequence Analysis | | | |
|---|---|---|---|
| **Selecting anti-RhD antibody (Ab)** | **Peptide sequence 10-mer Circular cys 10 amino acid cys** | **Peptide sequence 9-mer Linear 9 amino acids** | **Peptide sequence 6-mer Linear 6 amino acids** |
| IgG1-3 (i.e. full length IgG1 of Ab 3) | CGGMVDVYNGRC | | |
| Fab fragment of Ab 3 | CGGMVDVYNGRC | | |
| Fab fragment of Ab 3 | | MRYDYDILMAA (includes 2 amino acids AA from vector) | |
| Fab fragment of Ab 3 | | MRYDYDILM | |

Table 1   (continued)

| Results of the Sequence Analysis | | | |
|---|---|---|---|
| **Selecting anti-RhD antibody (Ab)** | **Peptide sequence 10-mer Circular cys 10 amino acid cys** | **Peptide sequence 9-mer Linear 9 amino acids** | **Peptide sequence 6-mer Linear 6 amino acids** |
| Rhophylac (affinity purified) | CKGFWDREWGLC | | |
| Rhophylac (affinity purified) | CGYWSAKWAVKC | | |
| Rhophylac (affinity purified) | | YFFPRGSGMAA (includes 2 amino acids AA from vector) | |
| Rhophylac (affinity purified) | | YFFPRGSGM | |
| Rhophylac (affinity purified) | | WHLSP (truncated peptide) | |
| Rhophylac (affinity purified) | | RHLSP (truncated peptide) | |
| IgG1-3 | | | QLIVMV |
| IgG1-3 | | | RLIVMV |

3.2 Specificity of ABP-IgG1-3 mimotope (cyclic 10-mer peptide)

The accompanying Figure 1 shows the ELISA results when the ABP-IgG1-3 mimotope (Mim) was immobilized on ELISA plates. The ABP-Myc refers to the control fusion protein without a peptide insert. In all cases there was no reactivity on the ABP-Myc protein alone. But there was a clear positive concentration dependent reaction of the antibodies IgG1-3 and IgG1-33. It should be noted that the selecting antibody for this mimotope was IgG1-3 but that IgG1-33 is very closely related to IgG1-3. In fact there are only 2 amino acid differences in the variable region of the IgG light chain between the 2 antibodies. Therefore it is not surprising that both these antibodies react with the ABP-IgG1-3 mimotope. However all other antibodies tested show no reactivity on the ABP-IgG1-3 mimotope.

Other antibodies tested:

Anti-Rh D33, anti-Rh D 52 and anti-Rh D 14 are disclosed in W097/49809. The sequence of the variable regions of the Fab fragments thereof is also deposited at the EMBL data bank as clones LD-6-33 (VH: AJ 005979; VL: AJ 005980), LD 1-52 (VH: Y08177; VL Y08193) and LD2-14 (VH: Y08187; VL: Y08248).

Anti-Rh D AD1, AD3, MS201, and S1 are monoclonal human antibodies either IgG or IgM class available from Biotest AG, Dreieich, Germany (AD1, AD3) Dade AG (MS 201) or Diagast AG (S1).

Anti-TTd (ST18) IgG is a human monoclonal IgG anti-tetanus toxoid antibody (Serum and Vaccine Institute, Bern).

Anti-alpha chain (LTMa15) antibody is a human recombinant IgG antibody reacting with the alpha chain of the high affinity receptor for IgE (Institute of Immunology and Allergy, Inselspital, Bern).

**Claims**

1. Peptides capable of binding to Rhesus D antibodies comprising:

(a)

(i) the amino acid sequence (I):

<div align="center">GGMVDVYNGR</div>

(ii) the amino acid sequence (I) wherein up to 3 amino acids are replaced by different amino acids or
(iii) an amino acid sequence which comprises at least 6 consecutive amino acids from (I);

(b)

(i) the amino acid sequence (II):

<div align="center">MRYDYDILM</div>

(ii) the amino acid sequence (II) wherein up to 3 amino acids are replaced by different amino acids or
(iii) an amino acid sequence which comprises at least 6 consecutive amino acids from (II);

(c)

(i) the amino acid sequence (III):

<div align="center">KGFWDREWGL</div>

(ii) the amino acid sequence (III) wherein up to 3 amino acids are replaced by different amino acids or
(iii) an amino acid sequence which comprises at least 6 consecutive amino acids from (III);

(d)

(i) the amino acid sequence (IV):

<div align="center">GYWSAKWAVK</div>

(ii) the amino acid sequence (IV) wherein up to 3 amino acids are replaced by different amino acids or
(iii) an amino acid sequence which comprises at least 6 consecutive amino acids from (IV);

(e)

(i) the amino acid sequence (V):

YFFPRGSGM

(ii) the amino acid sequence (V) wherein up to 3 amino acids are replaced by different amino acids or
(iii) an amino acid sequence which comprises at least 6 consecutive amino acids from (V);

(f)

(i) the amino acid sequence (VI):

WHLSP

(ii) the amino acid sequence (VII):

RHLSP

or
(iii) an amino acid sequence which comprises at least 4 consecutive amino acids from (VI) or (VII).

(g)

(i) the amino acid sequence (VIII):

QLIVMW

(ii) the amino acid sequence (IX):

RLIVMW

or
(iii) an amino acid sequence which comprises at least 4 consecutive amino acids from (VIII) or (IX)

2. Peptides according to claim 1 which have a length from 5 to 20 amino acids.

3. Peptides according to claims 1 or 2 which are incorporated into a vesicle or coupled to a carrier.

4. Peptides according to any one of claims 1-3 which are genetically fused to a recombinant polypeptide.

5. Peptides according to claim 4 wherein the recombinant polypeptide is a recombinant phage polypeptide.

6. Peptides according to claim 4 wherein the recombinant polypeptide is a pharmaceutically acceptable polypeptide.

7. Peptides according to any one of claims 1-6 coupled to a solid phase.

8. A pharmaceutical composition comprising as an active ingredient at least one peptide or polypeptide according to any one of claims 1-7.

9. The composition of claim 6 which is a diagnostic, prophylactic or therapeutic composition.

10. Use of peptides or polypeptides of any one of claims 1-7 for the manufacture of an agent for the diagnosis, therapy or prophylaxis of diseases associated with Rhesus D antigen.

11. Use according to claim 10 wherein the disease is hemolytic disease of the newborn (HDN) or idiopathic thrombocytopenic purpura (ITP).

**12.** Use of peptides or polypeptides of any one of claims 1-7 for the manufacture of an affinity reagent for anti-Rhesus D antibodies.

**13.** Use of claim 12 wherein anti-Rhesus D antibodies are purified or removed from body fluids or immunoglobulin preparations.

**14.** A nucleic acid encoding the peptide or polypeptide of any one of claims 1 to 7.

**15.** A vector comprising at least one copy of a nucleic acid according to claim 14, preferably in operative linkage to an expression control sequence.

**16.** A cell comprising the nucleic acid of claim 14 or the vector of claim 15.

**17.** A pharmaceutical composition comprising as an active ingredient at least one nucleic acid or vector according to claim 14 or 15.

**18.** A method of preparing the peptide or polypeptide of any one of claims 1 to 7 comprising culturing the cell of claim 16 under suitable conditions wherein the peptide or polypeptide is produced and obtaining the peptide or polypeptide from the cell or the culture medium.

**19.** A method for identifying peptides having immunologic properties of Rhesus D protein epitopes comprising subjecting an antibody or an antibody fragment recognizing an epitope of Rhesus D protein to several panning rounds with a phage display library and identifying immunogenic peptide sequences wherein said peptide sequences are mimotopes which differ in their amino acid sequence from the amino acid sequence of Rhesus D protein.

**20.** Peptides having immunologic properties of Rhesus D protein epitopes which differ in their amino acid sequence from the amino acid sequence of Rhesus D protein obtainable by the method of claim 19.

## Fig. 1

ABP-IgG1-3-Mim-ELISA mit diversen Antikörpern vom 27.10.1999

Fig. 1

ABP-IgG1-3-Mim-ELISA mit diversen Antikörpern vom 27.10.1999

16

European Patent Office

## EUROPEAN SEARCH REPORT

Application Number

EP 99 12 2858

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE ENTREZ GENPEPT 'Online! Accession Number AAB24655, 8 May 1993 (1993-05-08) ROSENBLATT JD, ET AL: "Rig homolog" XP002134527 * abstract * | 1,14-16, 20 | C07K7/06 A61K38/08 G01N33/53 C12N15/11 A61K31/7088 C12N1/21 |
| D,A | WO 97 49809 A (ROTKREUZSTIFTUNG ZENTRALLAB ;MIESCHER SYLVIA (CH); VOGEL MONIQUE ()) 31 December 1997 (1997-12-31) * the whole document * | 1-20 | |
| D,A | MIESCHER S, ET AL: "Sequence and Specificity Analysis of Recombinant Human Fab Anti-Rh D Isolated by Phage Display" VOX SANGUINIS, vol. 75, no. 4, December 1998 (1998-12), pages 278-287, XP002134525 * the whole document * | 1-20 | |
| D,A | LE VAN KIM C, ET AL.: "Molecular cloning and primary structure of the human blood group RhD polypeptide" PROCEEDINGS OF THE NATIONAL ACADEMY OF SCIENCES OF THE USA, vol. 89, November 1992 (1992-11), pages 10925-10929, XP002134526 * the whole document * | 1-20 | **TECHNICAL FIELDS SEARCHED (Int.Cl.7)** C07K |
| A | STOTT L-M, ET AL: "Mapping Alloreactive T Cell Epitopes on the Rhesus D Protein" BLOOD, vol. 92, 15 November 1998 (1998-11-15), page 25A XP000882608 * abstract * | 1-20 | |

-/--

The present search report has been drawn up for all claims

| Place of search | Date of completion of the search | Examiner |
|---|---|---|
| MUNICH | 31 March 2000 | Bilang, J |

European Patent Office

**EUROPEAN SEARCH REPORT**

Application Number

EP 99 12 2858

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | GRIEVER G, ET AL.: "Delineation of a first T cell epitope in the Rhesus D polypeptide" BLOOD, vol. 90, 15 November 1997 (1997-11-15), page 58B XP000882607 * abstract * | 1-20 | |
| | | | TECHNICAL FIELDS SEARCHED (Int.Cl.7) |

The present search report has been drawn up for all claims

| Place of search | Date of completion of the search | Examiner |
|---|---|---|
| MUNICH | 31 March 2000 | Bilang, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 99 12 2858

This annex lists the patent family members relating to the patent documents cited in the above–mentioned European search report.
The members are as contained in the European Patent Office EDP file on
The European Patent Office is in no way liable for these particulars which are merely given for the purpose of information.

31–03–2000

| Patent document cited in search report | Publication date | Patent family member(s) | | Publication date |
|---|---|---|---|---|
| WO 9749809 A | 31–12–1997 | AU | 3342397 A | 14–01–1998 |
| | | BG | 103016 A | 31–08–1999 |
| | | BR | 9709958 A | 10–08–1999 |
| | | CA | 2258494 A | 31–12–1997 |
| | | CZ | 9803959 A | 12–05–1999 |
| | | EP | 0920509 A | 09–06–1999 |
| | | HU | 9902605 A | 29–11–1999 |
| | | NO | 986088 A | 24–02–1999 |
| | | PL | 330794 A | 07–06–1999 |
| | | SK | 176198 A | 12–07–1999 |

EPO FORM P0459

Annex to the application documents - subsequently filed sequences listing

SEQUENCE LISTING

<110> ZLB Zentrallaboratorium Blutspendedienst SRK

<120> Rhesus D specific peptide sequences

<130> 21595pep

<140> 99122858.6
<141> 1999-11-17

<160> 9

<170> PatentIn Ver. 2.1

<210> 1
<211> 10
<212> PRT
<213> Artificial Sequence

<220>
<223> Description of Artificial Sequence: peptides
      capabale of binding to Rhesus D  antibodies

<400> 1
Gly Gly Met Val Asp Val Tyr Asn Gly Arg
  1               5               10


<210> 2
<211> 9
<212> PRT
<213> Artificial Sequence

<220>
<223> Description of Artificial Sequence: peptides
      capable of binding to Rhesus D antibodies

<400> 2
Met Arg Tyr Asp Tyr Asp Ile Leu Met
  1               5


<210> 3
<211> 10
<212> PRT

<213> Artificial Sequence

<220>
<223> Description of Artificial Sequence: peptides
      capable of binding to Rhesus D antibodies

<400> 3
Lys Gly Phe Trp Asp Arg Glu Trp Gly Leu
  1               5                   10


<210> 4
<211> 10
<212> PRT
<213> Artificial Sequence

<220>
<223> Description of Artificial Sequence: peptides
      capable of binding to Rhesus D antibodies

<400> 4
Gly Tyr Trp Ser Ala Lys Trp Ala Val Lys
  1                5                   10


<210> 5
<211> 9
<212> PRT
<213> Artificial Sequence

<220>
<223> Description of Artificial Sequence: peptides
      capable of binding to Rhesus D antibodies

<400> 5
Tyr Phe Phe Pro Arg Gly Ser Gly Met
  1               5


<210> 6
<211> 5
<212> PRT
<213> Artificial Sequence

<220>

<223> Description of Artificial Sequence: peptides
      capable of binding to Rhesus D antibodies

<400> 6
Trp His Leu Ser Pro
  1            5


<210> 7
<211> 5
<212> PRT
<213> Artificial Sequence

<220>
<223> Description of Artificial Sequence: peptides
      capable of binding to Rhesus D antibodies

<400> 7
Arg His Leu Ser Pro
  1            5



<210> 8
<211> 6
<212> PRT
<213> Artificial Sequence

<220>
<223> Description of Artificial Sequence: peptides
      capable of binding to Rhesus D antibodies

<400> 8
Gln Leu Ile Val Met Trp
  1            5



<210> 9
<211> 6
<212> PRT
<213> Artificial Sequence

<220>
<223> Description of Artificial Sequence: peptides
      capable of binding to Rhesus D antibodies

```
<400> 9
Arg Leu Ile Val Met Trp
 1           5
```